# EUROPEAN PATENT APPLICATION

(11) **EP 1 291 415 A1**
(43) Date of publication of application: **12.03.2003**
(21) Application number: 01934424.1
(22) Date of filing: 30.05.2001
(51) Int. Cl.: C12N 5/08

(54) **METHOD OF PREPARING SMALL HEPATOCYTES PRESERVABLE IN FREEZE-DRIED STATE AND METHOD OF PRESERVING THE SAME IN FREEZE-DRIED STATE**

(30) Priority: 01.06.2000 JP 2000164158
(71) Applicant: Hokkaido Technology Licensing Office Co., Ltd., Sapporo-Shi, Hokkaido 060-0807 (JP)
(72) Inventor: MITAKA, Toshihiro, Kiyota-Ku, Sapporo-Shi, Hokkaido004-0863 (JP)
(74) Representative: Hallybone, Huw George
(86) International application number: JP0104555
(87) International publication number: WO01092481

(57) **Abstract**

A method of preparing small hepatocytes is provided. The method is suitable for cryopreservation wherein the hepatic function and proliferation ability of the small hepatocytes is retained. A method of cryopreserving thus prepared small hepatocytes and the cryopreserved small hepatocytes having hepatic functions and proliferation ability are also provided. According to the present invention, small hepatocytes are cultured using a medium supplemented with nicotinamide to form colonies of small hepatocytes in which the small hepatocytes are encompassed by nonparenchymal cells, and then the formed colonies are dissociated from culture dishes as small hepatocytes aggregate by non-enzymatic treatment, suspended in a cryopreservation solution and are cryopreserved.

## Description

### Background of the Invention

The present invention relates to a method of preparing cryopreservable small hepatocytes and a method of cryopreserving the small hepatocytes.

Liver and hepatocytes possess multiple functions so that they are compared to a chemical factory in the living body. For example, more than 90% of serum proteins are produced by hepatocytes, which have a detoxification function of metabolizing toxic substances that were incorporated into or produced in the living body. Thus researchers in various institutions have been attempted to culture hepatocytes, detect harmful substances (bio-sensor) and allow to produce substances needed for human being in vitro using the functions of hepatocytes. In order to supply hepatocytes used for such investigations, it is necessary to isolate mature hepatocytes for every experiment, because there are currently no cell lines possessing the differentiated functions of hepatocytes. In such circumstances, the number of obtainable cells depends on the number of hepatocytes in individuals, because a method of proliferating mature hepatocytes maintaining the functions of hepatocytes has not been established.

A continuous large supply of cells having a major part of the functions of mature hepatocyte has been desired in the development of built-in type artificial livers. Thus, it has been an important issue to develop a method of cryopreserving hepatocytes, preserving them for a long time and reusing hepatocytes from animals including human being, which has been investigated worldwide. However, there are previously only a few reports wherein cryopreserved hepatocytes had been lively adhered to the culture dishes after being thawed and had been able to maintain about 70 to 80% of their functions as hepatocytes only for a short period. Furthermore, in these reports, the thawed hepatocytes had very poor proliferation ability and died within a short period.

On the other hand, the inventors reported a cell population in the liver tissue which exhibited similar phenotypes for makers of mature hepatocytes such as albumin, transferrin, cytokeratin (CK) 8 or CK18 and had the ultrastructural characteristics for mature hepatocyte, while the population was composed of small cells having high proliferation ability. These cells were named "small hepatocyte" (Mitaka T. et al., Hepatology, 16, 440-447 (1992)).

While such studies have been made, there have been no reports on the methods of preparing cells which could be cryopreserved by a simple way and which could proliferate after being thawed and retain or enhance the functions as hepatocytes for a long time, and there have been also no reports on the methods of cryopreservation of such cells.

### Summary of the Invention

The object of the present invention is to provide a method of preserving small hepatocytes for a long period remaining the functions as hepatocytes and proliferation ability thereof. Particularly, the object of the present invention is to provide a method of cryopreservation of small hepatocytes which can retain the functions as hepatocytes and proliferation ability.

Another object of the present invention is to provide a method of preparing small hepatocytes wherein said method is suitable to the cryopreservation which can retain the functions of the small hepatocytes as hepatocytes and proliferation ability thereof.

The further object of the invention is also to provide small hepatocytes that have been prepared and cryopreserved as aforementioned.

As described above, the inventors have already found the small hepatocytes which exhibit high proliferation ability and which have a large parts of the functions of mature hepatocytes. The inventors found that the small hepatocytes could be cryopreserved and could have high proliferation ability and retain the hepatocyte functions after being thawed, when they had been cultured under a certain condition.

Thus, the present invention is a method of preparing small hepatocytes for cryopreservation which comprises;
(i) isolating hepatocytes from a liver;
(ii) separating the hepatocytes into a light fraction which is enriched with nonparenchymal cells and contains less parenchymal cells and a heavy fraction which is enriched with parenchymal cells, and recovering the light fraction; and,
(iii) culturing cells contained in the light fraction using a medium supplemented with nicotinamide to from a colony of small hepatocytes encompassed by nonparenchymal cells.

The present invention is also a method of preparing a small hepatocyte for cryopreservation which comprises;
(i) dissociating small hepatocytes which are encompassed by nonparenchymal cells and are forming a colony from a culture dish by reacting or without reacting an enzyme and recovering the small hepatocytes; and
(ii) culturing the recovered small hepatocytes successively using a medium supplemented with nicotinamide to from a colony of small hepatocytes encompassed by nonparenchymal cells.

The present invention is also a method of cryopreserving small hepatocytes which comprises;
(i) dissociating small hepatocytes which have been cultured to form a colony encompassed by nonparenchymal cells from a culture dish as a small hepatocyte aggregate;
(ii) Optionally, washing the dissociated small hepatocyte aggregate with a medium or a buffer solution;
(iii) suspending the small hepatocyte aggregate in a cryopreservation solution;
(iv) transferring the suspended small hepatocyte aggregate into a freezing tube and freezing said suspended small hepatocyte aggregate; and
(v) cryopreserving the frozen small hepatocyte aggregate.

Particularly, in the method of the present invention, the freezing step was conducted in two steps wherein small hepatocytes are frozen at about - 10°C to about -30°C, followed by freezing at about -50°C to -90°C or in liquid nitrogen, and cryopreserving the cells at -50°C to -90°C or in liquid nitrogen.

### Brief Description of the Drawings

Figure 1 is the schematic illustration showing how cells, such as liver epithelial cells, stellate cells or sinusoidal endothelial cells will encompass the small hepatocytes during the small hepatocytes form a colony.
Figure 2 is the schematic illustration showing the small hepatocyte aggregate present in the cryopreservation system after dissociating the small hepatocyte aggregate from a culture dish which was formed by the encompassment of small hepatocytes by liver epithelial cells, stellate cells or sinusoidal endothelial cells.
Figure 3 is the result of periodical determination of the area of thawed small hepatocyte colonies when the colonies have been cryopreserved at -80°C for six (6) months. Each value of longitudinal axis is relatively indicated based on the average colony area at day 1 of culture as 100%. The horizontal axis indicates the days from the start of culture, after thawing. The bars indicate the standard deviation (SD).
Figure 4 shows the production ability of albumin of the small hepatocytes which has been cryopreserved for six (6) months. The horizontal axis indicates the days from the start of culture, after thawing. The bars indicate the standard deviation (SD).

### Description of the Preferred Embodiments

A small hepatocyte is used for the method of the present invention. The term "small hepatocyte" as used herein does not merely mean a small cell which is derived from a liver, but it means a small cell which may be isolated according to the following procedures or the similar procedures and which exhibits substantially similar phenotypes to a mature hepatocyte regarding the markers such as albumin, transferrin, cytokeratin (CK) 8 and CK 18 and has the ultrastructural features as a hepatocyte. These cells were found by the inventors, the details of which were described in Mitaka, T. et al., Hepatology, 16, 440-447 (1992) and Mitaka, T., Hepatology 29, 111-125 (1999).

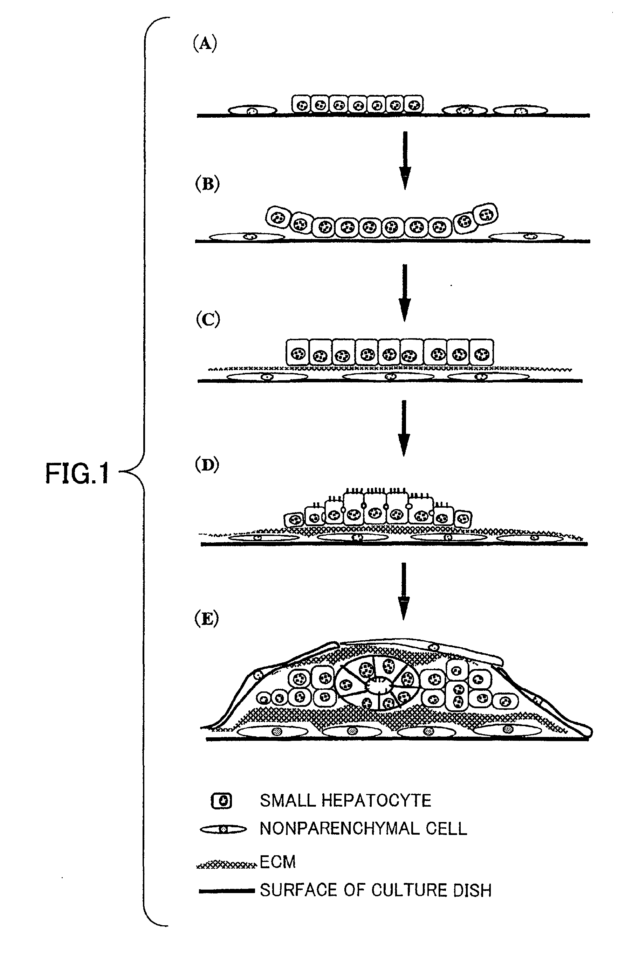

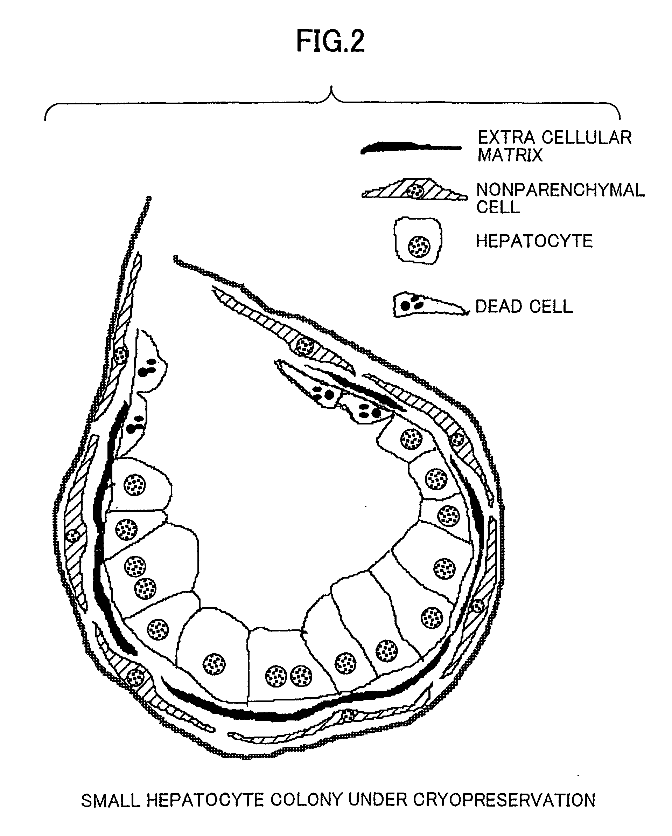

Additionally, a "small hepatocyte aggregate" does not merely refer to the status where many cells were loosely bound each other such as in spheroids, but it means the cell aggregate which has the particular structure formed according to the present methods. Namely, a "small hepatocyte aggregate" refers to a particular structure formed during the dissociation of the colony of small hepatocytes from a culture dish at the time when nonparenchymal cells such as liver epithelial cells, stellate cells and sinusoidal endothelial cells surround the colony or when all or a part of the dish-side of the small hepatocyte colony is covered by nonparenchymal cells, after the small hepatocytes adhere on the culture dish and proliferate to the two-dimensional colony (Fig. 1B and 1C). Thus, a "small hepatocyte aggregate" is a mass of cells, which has the specific structure where nonparenchymal cells such as liver epithelial cells, stellate cells and sinusoidal endothelial cells encompass the colony of small hepatocytes as a central part of the mass, and which is formed when the small hepatocyte colony is dissociated from a culture dish together with the peripheral cells (Fig. 2). When it is apparent in the context, a "small hepatocyte aggregate" may be also simply referred to as a "cell aggregate".

The small hepatocytes used in the present invention may be prepared as described below. Liver-derived cells can be obtained by treating liver tissue from human being or other animals with a solution containing a collagenase and the like. For this purpose, a conventional liver perfusion method may be used. The obtained cell suspension may be passed through a mesh of an appropriate size to remove tissue debris such as undigested tissue residues and the like. Although the cell suspension can be directly used to obtain the small hepatocyte colonies, it is preferred to remove parenchymal cells and undesired tissue residues to a certain extent before culturing the cells. Parenchymal cells can be removed by low speed centrifugation as described below. The low speed centrifugation is the condition which is sufficient to separate the light fraction which is enriched with nonparenchymal cells from the fraction mainly containing parenchymal cells and undesired tissue residues, preferably the condition sufficient to separate the light fraction which is enriched with small hepatocytes and nonparenchymal cells and which is substantially free of parenchymal cells from the fraction mainly containing parenchymal cells and undesired tissue residues. When the cells obtained from a liver according to the described method are fractionated under such conditions, the small hepatocytes may be obtained in the light fraction in larger quantities. However, it is not necessary to completely separate small hepatocytes from nonparenchymal cells, because it is necessary to culture them in a mixed culture. Particularly, the cell suspension can be separated into a heavy fraction which mainly contains parenchymal cells and a light supernatant fraction which mainly contains relatively light cells including nonparenchymal cells such as stellate cells, Kupffer's cell and sinusoidal endothelial cells by, for example, centrifuging at 50 x g for one (1) minute. Larger amount of the small hepatocytes may be obtained in the supernatant fraction under this centrifugation condition.

The centrifugation is preferably performed at about 50 x g for not more than 1 minute, because the ratio of precipitated small hepatocytes may increase as the speed or the time of centrifugation increases, although the ratio of parenchymal cells in the supernatant fraction decreases. Parenchymal cells and undesired tissue debris and the like may be further removed by repeatedly centrifuging, precipitating and suspending the supernatant fraction. More specifically, for example, the supernatant fraction may be centrifuged at 50 x g for 5 minutes and the precipitate is suspended in an appropriate medium and further centrifuged at 50 x g for 5 minutes. The precipitate is suspended in a similar medium, centrifuged again at 50 x g for 5 minutes and the precipitated cells are suspended in a fresh medium. The cell number in the cell suspension may be determined to adjust the cell density required for the subsequent culture or the treatments. Generally, the density is adjusted to 1 x 10⁵ to 5 x 10⁵ cells/ml.

The thus prepared cells may be cultured in a basal medium containing serum, nicotinamide, vitamin C, antibiotics, growth factors and other additives commonly used for cell culture, for example Dulbecco's modified Eagle's medium supplemented with these substances, at 37°C. The medium used for the present invention contains nicotinamide and preferably further contains vitamin C, growth factors, DMSO and the like to accelerate colony formation. Vitamin C is usually added as ascorbic acid 2-phosphate preferably at a concentration of 0.1 mM to 1.0 mM, more preferably 0.5 to 1.0 mM, and nicotinamide is used at a relative high concentration, preferably 1 to 20 mM and more preferably 5 to 10 mM. As the growth factors, epidermal growth factor (EGF), hepatocyte growth factor (HGF), or transforming growth factor α (TGF α) and the like may be used. TGF α is particularly preferred. When TGF α is added, it is preferably to use it at a concentration of 1 µg/l to 100 µg/l, more preferably 5 µg/l to 50 µg/l. DMSO is preferably added at a concentration of about 0.1 to about 2 % (v/v), more preferably about 0.5 % to about 1.5% (v/v) on and after day 4 after starting the culture. The medium is changed almost every other day, usually 3 times a week.

Regarding the container for culturing the small hepatocytes used in the present invention, any culture dish conventionally used for cell culture may be used. Generally, collagen-coated dishes are used for the culture of adherent cells. For example, the culture dishes of various sizes which are coated by collagen derived from bovine dermis or rat tail are commercially available. It is also possible to prepare such dishes, if necessary. It is possible to use such dishes for the culture of the small hepatocytes used in the present invention, but it is preferable to use dishes which are not coated by collagen because the cells can be dissociated from the dishes in the mild condition more easily when the extracellular matrix is less. In the present invention, the culture may be preferably started at relatively high cell density, particularly at about 4 x 10⁵ to 9 x 10⁵ cells per 60 mm culture dish. A conventional 5% CO₂ incubator may be used for the culture. The range of CO₂ concentration and the temperature are not essential as long as they are within the range which is acceptable for conventional cultured cells.

When the small hepatocytes are cultured under such conditions, they start to form colonies and they form the distinguished colonies encompassed by nonparenchymal cells in about 1 to about 2 weeks. These colonies may be continuously further cultured, but it is preferable to use the colonies at the stage where nonparenchymal cells surround the peripheries of cell agglomerations formed by the small hepatocytes or at the stage where nonparenchymal cells and ECM (extracellular matrix) invade into the culture dish-sides of the small hepatocytes to form the structure where the culture dish-sides of the small hepatocytes are completely or partially covered by nonparenchymal cells and ECM (Fig 1B or 1C). As used herein, when nonparenchymal cells "encompass" the small hepatocytes, it includes the case where nonparenchymal cells surround the periphery of the cell agglomerate formed by the small hepatocytes and/or the case where nonparenchymal cells invade into the culture dish-sides of the small hepatocytes to cover all or a part of the culture dish-sides of the small hepatocytes (Fig. 1B and C). The cells at this stage can be visually confirmed easily because of the formation of colonies. Excessive long-term culture makes it difficult to dissociate the cells from culture dishes and may tend to reduce the proliferation ability when the cells are thawed after cryopreservation. Additionally, it is preferable to use the small hepatocytes at the stage before the stage where large mature hepatocytes are beginning to appear in the small hepatocyte colonies (Fig. 1D) or the formation of hepatoid tissues become apparent (Fig. 1E), namely before passing not more than about 1 month from the starting of the culture. More specifically, for cryopreservation it is preferable to use the colonies of day 8 to day 20, more preferably day 10 to day 14 after starting culture.

The formed colonies can be disaggregated into individual cells in the presence of metal chelators and/or enzymes and the cells can be successively subcultured to re-form colonies. However, in this case, a longer period may be generally required to re-form colonies which are in an appropriate condition suitable for the method of cryopreservation of the present invention, because the damages to the cells may be extensive. Thus, it is preferable to dissociate the colonies from culture containers by non-enzymatic procedures as described below in the successive subculture. In any cases, a medium containing nicotinamide, vitamin C, growth factors, DMSO and the like may be used as is used for obtaining small hepatocytes directly from a liver. The concentrations of nicotinamide, vitamin C, growth factors and DMSO may be the same as they are used for obtaining small hepatocytes directly from a liver.

For cryopreservation according to the present invention, the cell aggregations are dissociated under the mild condition so that the aforementioned structure is not destroyed. For example, it is preferable to dissociate the cells from culture dishes non-enzymatically using metal chelators or various non-enzymatic dissociation agents. The metal chelators which can be used may be any metal chelators having less cytotoxicity. The chelators which are generally used for dissociating treatment of adherent cells, for example, EDTA, EGTA or the salts thereof may be used at the concentration generally used for them. The sodium salts of EDTA are particularly preferred and are preferably use at a concentration of 0.01% to 0.05% (w/v), more preferably at a concentration of 0.01% to 0.02% (w/v). For sodium salts of EGTA, it is preferable to use it at a concentration of about 0.5 mM. The treatment time may be as short as the time required to rinse the cells (a few seconds), but is preferably about 30 seconds to about 10 minutes, more preferably about 1 minute to about 5 minutes. The treatment time may be preferably as short as possible, because the treatment for a longer time would extensively damage the cells.

Among non-enzymatic dissociation agents, a solution of Hank's buffer (pH 7.3 - 7.5) without Ca and Mg supplemented with EDTA, glycerol, sodium citrate and the like may be used. For example, a prepared non-enzymatic cell dissociation agent can be commercially available from Sigma Chemical Co., which is marketed as "Cell Dissociation Solution". More specifically, for example, phosphate buffer solution containing about 0.02% EDTA is poured onto the cells. After standing them for a few minutes, EDTA solution is removed, and a non-enzymatic dissociation solution such as Cell Dissociation Solution is poured, then they are remained for about 5 to about 30 minutes, preferably about 10 to 15 minutes at 37°C. To minimize the damage to the cells, the time of treatment with the non-enzymatic dissociation agent is preferably as short as possible, such as in the metal chelate treatment. Then, the colonies containing small hepatocytes are dissociated from the culture dishes by mildly pipetting to minimize the damage to the cells. The colonies are isolated as the aggregates described above. The aggregates are optionally washed by repeatedly centrifuging and suspending, and the aggregates are finally suspended in an appropriate medium and the like. A cryopreservation solution for suspending the cells for cryopreservation may be a solution which is commonly used for the cryopreservation of cultured cells, which may contain a medium, DMSO and various agents for cryopreservation as described below.

The cell aggregate is an agglomerate of cells, which has the special structure where nonparenchymal cells such as liver epithelial cells, stellate cells and sinusoidal endothelial cells encompass the colony of the small hepatocytes as a central part of the agglomerate. Particularly, the cell aggregate takes the form in which aforementioned nonparenchymal cells envelope the small hepatocytes within, when the aggregate is dissociated from the culture dish. In other words, the small hepatocytes prepared according to the present invention form the structure which is protected from freezing agents and external stimulation under the favor of peripheral nonparenchymal cells and extra cellular matrices (Fig. 2). It is possible to form such structures according to the present method of preparing the small hepatocytes, which is essential for the method of cryopreservation of the present invention.

To the thus prepared cell suspension, DMSO is added to a concentration of, for example, 5% to 10% to prepare the cell suspension for cryopreservation. It is preferable to standing the suspension for about 10 minutes to about 20 minutes at room temperature so that the cells acclimatize to DMSO. Aliquots of the cell suspension, for example, 0.2 ml to 2 ml per tube, are transferred to freezing tubes, and then they are cryopreserved. The freezing may be carried out rapidly by immediately placing the tubes in liquid nitrogen. When a preservation agent such as CellBanker and Kainos is used, the suspension can be more easily cryopreserved by freezing in a freezer at -80°C and directly cryopreserved. However, it is preferable to freeze more mildly according to the temperature shift or temperature gradient which is generally used for cryopreservation of cultured cells so that the proliferation ability is more highly retained after thawing. For example, the cell suspension may be left at about -20°C to about -30°C without agitating, then -50°C to -90°C, preferably -70°C to -90°C or in liquid nitrogen before cryopreservation. A programmable freezer is also commercially available to accomplish the temperature shift suitable for cryopreservation of the cells. Particularly, it is possible to cryopreserve the cells for a long period at -50°C to -90°C, preferably at -70°C to -90°C, if a preservation agent such as CellBanker or Kainos is additionally used. Generally, the small hepatocytes are preferably preserved in liquid nitrogen, if such additional preservation agents are not used.

The thus cryopreserved small hepatocytes maintain their proliferation ability and their functions as hepatocytes at least for more than one year. The cryopreserved small hepatocytes may be thawed at any time to obtain the desired amount of the cells. It is preferable that the cryopreserved small hepatocytes are rapidly thawed, the method of which are well known to those skilled in the art. For example, the thawing may be rapidly carried out in a water bath at 37°C. The thawed cells are cultured under the conditions as described above. Optionally, the cells may be washed by repeatedly centrifuging and suspending them, before starting the culture. Various markers may be used to confirm that the cultured small hepatocytes have some of the functions of hepatocytes. Such markers include, besides albumin which is secreted in the medium, transferrin, production of urea, amount of glycogen, cytochrome P450 and the like. These markers may be confirmed by ELISA or Western blot analysis of the medium or the cell extract, or by directly immunostaining of the cells. The small hepatocytes can be distinguished from other cells in that they exhibit the markers of mature hepatocytes such as albumin, CK8, CK18, glycogen or peroxisome but they do not exhibit the markers of biliary epithelial cells such as CK7 or CK19. For example, oval cell, which is known to be a kind of hepatic stem cells, exhibits the markers of biliary epithelial cells such as CK7 and CK19 but it does not exhibit the markers of mature hepatocytes such as glycogen or peroxisome.

Required amount of the cryopreserved small hepatocytes can be thawed at any time and the cells can be used for various purposes such as various cell-embedded type artificial livers. The small hepatocytes which has been prepared and cryopreserved according to the present invention exhibit vigorous proliferation ability even after being thawed and the cell number increases up to 100-fold in about 3 weeks. Additionally, since the small hepatocytes may form hepatoid tissues by long-term culture (1 month or more) or by coating them with ECM, they are also useful as a model system for liver regeneration. The thus matured small hepatocytes may be also used for the study of liver functions as an alternative for experimental animal. Furthermore, it is possible to form a liver tissue by transplanting the cultured small hepatocytes directly into the liver *in vivo.* Particularly, the small hepatocytes from human being can be used for this purpose. It is also understood that the small hepatocytes, which can be cryopreserved, are useful as an experimental material in the molecular study of hepatocyte functions and in the study of cell biology.

### Examples

### Example 1. Isolation of small hepatocytes

A liver of mature rat (10 to 15 weeks old) was perfused through portal vein by Hank's solution supplemented with 0.2 mM EGTA without Ca and Mg according to the similar method of Seglen. Hank's solution was perfused for 4 minutes at a flow rate of 40 ml/min, and then Hank's solution containing 0.02% collagenase (Yakult Co.) was perfused for 10 minutes at a flow rate of 20 ml/min. The hepatocytes were washed into a beaker from the digested liver according to the ordinary method. The cell suspension was passed through a 70µm-mesh filter and centrifuged for 1 minute at 50 x g. The supernatant was collected and centrifuged for 5 minutes at 50 x g. The precipitated cells were washed with a medium (Leibovitz L-15, 10% bovine serum, 10⁻⁷M dexamethasone, 0.5 µg/ml insulin and antibiotics) and were centrifuged for 5 minutes at 50 x g. The similar procedure was repeated once more and the suspension was centrifuged again for 5 minutes at 50 x g. The precipitated cells were suspended in the fresh medium to prepare small hepatocytes. The number of viable cells was counted to adjust the cell density to 1.0- 2.0 x 10⁵ cells/ml.

### Example 2. Method of culturing the small hepatocytes

The cells prepared as described in Example 1 were plated on 60-mm dishes at about 6 x 10⁵ cells per dish. After incubated for 3 hours in an air incubator without agitation, the medium was replaced. The medium based on Dulbecco's modified Eagle's medium was used as described below. The cells were cultured in a 5% CO₂ incubator at 37°C and the medium was replaced every other day (3 times a week). After culturing for about 2 weeks, many colonies of hepatocytes were observed.

### Medium for culture of small hepatocytes

| Dalbecco's modified Eagle's medium (GIBCO Laboratories) | |
|---|---|
| + 20 mM HEPES | (Dojindo) |
| + 25 mM NaHCO₃ | (Katayama Chemical Co.) |
| + 30 mg/l proline | (Sigma Chemical Co.) |
| + 0.5 mg/l insulin | (Sigma Chemical Co.) |
| + 10⁻⁷ M dexamethasone | (Sigma Chemical Co.) |
| + 10% FBS | (Hyclone Laboratories, Inc.) |
| + 10 mM nicotinamide | (Katayama Chemical Co.) |
| + 1 mM L-ascorbic acid phosphate | (Wako Pure Chemical Inc.) |
| + antibiotics | |
| ( + 1 % dimethylsulfoxide* | (Aldrich) ) |

| | |
|---|---|
| * DMSO was added to the culture medium on and after day 4 medium exchange. | |

### Example 3. Cryopreservation procedure of cells

About 2 weeks after starting the culture, when many colonies of small hepatocytes appeared, the cells were dissociated from the dishes and were cryopreserved according to the following procedure.

The cells were washed twice with sterile phosphate buffer. Then, the cells were immersed in a phosphate buffer containing 0.02% EDTA. After standing a few minutes without agitating, the phosphate buffer was aspirated. One ml of enzyme-free Cell Dissociation Solution (Sigma) was introduced in the dishes and the dishes were left for 15 minutes at 37°C.

The cells were carefully dissociated from the dishes by gently pipetting not to damage the cells. The cells were collected into a centrifuge tube and were centrifuged for 5 minutes at 50 x g. After the supernatant was aspirated, the cells were washed with the medium containing 10% serum and were centrifuged again. After the supernatant was aspirated, CellBanker was added at 2 dishes/ml and the cells were gently suspended.

The thus prepared cell suspension was dispensed in 1 ml of aliquots into freezing tubes, and the tubes were kept in the freezer for 1 hour at - 30°C, and then moved to a -80°C freezer.

### Example 4. Culture of thawed small hepatocytes

The small hepatocytes which had been frozen as described in Example 3 and cryopreserved for 6 months were thawed and cultured after removing anti-freezing agents.

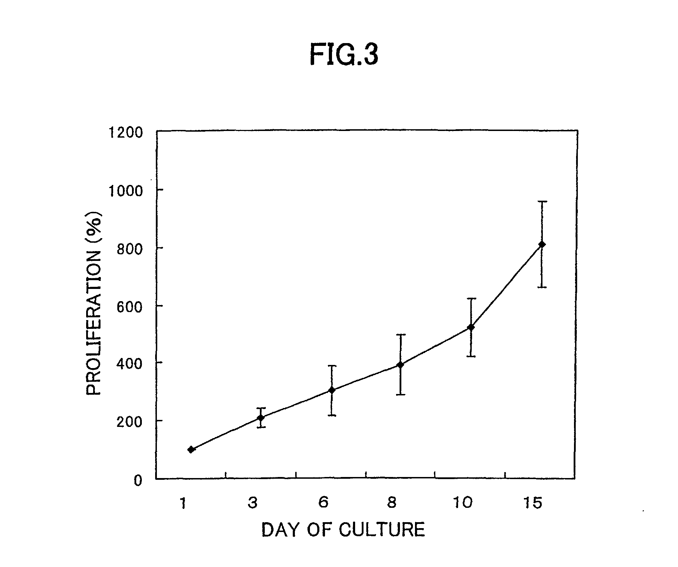

The freezing tubes containing the frozen cells were immersed in a 37°C water bath for rapid thawing. The cells contained in one freezing tube were transferred into a centrifuge tube containing 10 ml of medium. The cells were precipitated by centrifuging the tube for 5 minutes at 50 x g. After aspirating the supernatant, 18 ml of medium was added to the tube and divided into 3 ml aliquots in 60-mm culture dishes. The cells were incubated in a 5% CO₂ incubator at 37°C for 24 hours, and then the medium was replaced. The medium was further replaced every other day and the areas of the formed colonies are determined as an index for proliferation ability (Fig. 3).

The individual area of 30 colonies per dish was determined at the time of culture indicated in Fig. 3 using 3 dishes. The determined value was the average of values obtained from 3 dishes. Each measured value is shown in Fig. 3 by defining the average area of the colony at day 1 of culture as 100%. As shown in Fig. 3, it was demonstrated that the small hepatocytes which had been cryopreserved according to the present invention had potent proliferation ability even after being thawed.

### Example 5. Functional evaluation of the thawed small hepatocytes

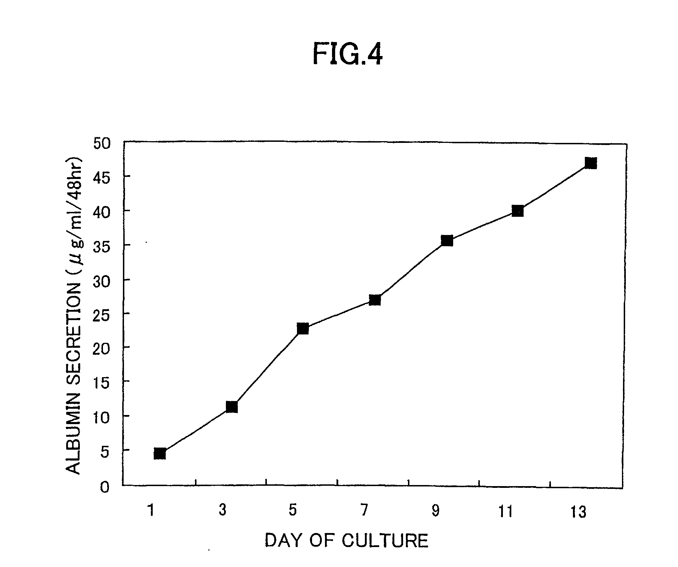

The cryopreserved cell which had been preserved for 6 months were thawed and cultured as described in Example 4 and the albumin production was examined 3 to 30 days after the culture was started. The amount of secreted albumin in the medium was determined by ELISA. The results obtained form 3 dishes were averaged and the standard deviation (SD) was also calculated to make a graph. The results are shown in Fig. 4. As is apparent from Fig. 4, the frozen small hepatocytes cultured and prepared according to the present invention had a significant ability of albumin production, and thus it was demonstrated that they did not loose the functions as hepatocytes during cryopreservation.

According to the present invention, it is possible to provide the desired amount of the small hepatocytes at any time which have the functions of hepatocytes and which can proliferate. It is also possible to cryopreserve the small hepatocytes for a long period retaining their functions as hepatocytes and the proliferation ability. The small hepatocytes prepared according to the present invention can be used for an artificial liver and can form liver tissue by transplanting them into fat tissue or liver. It is also possible to use the small hepatocytes for monitoring liver toxicity or liver function as an alternative for an experimental animal, after thawing and maturating the cryopreserved small hepatocytes in the culture dishes at any time.

## Claims

1. A method of preparing small hepatocytes for cryopreservation comprising;
(i) isolating hepatocytes from a liver;
(ii) separating the hepatocytes into a light fraction which is enriched with nonparenchymal cells and contains less parenchymal cells and a heavy fraction which is enriched with parenchymal cells, and recovering the light fraction; and,
(iii) culturing cells contained in the light fraction using a medium supplemented with nicotinamide to from a colony of small hepatocytes encompassed by nonparenchymal cells.

2. A method of preparing small hepatocytes for cryopreservation comprising;
(i) dissociating small hepatocytes which are encompassed by nonparenchymal cells and are forming a colony from a culture dish by reacting or without reacting an enzyme and recovering the small hepatocytes; and
(ii) culturing the recovered small hepatocytes successively using a medium supplemented with nicotinamide to from a colony of small hepatocytes encompassed by nonparenchymal cells.

3. A method of cryopreserving small hepatocytes comprising;
(i) dissociating small hepatocytes which have been cultured to form a colony encompassed by nonparenchymal cells from a culture dish as a small hepatocyte aggregate;
(ii) Optionally, washing the dissociated small hepatocyte aggregate with a medium or a buffer solution;
(iii) suspending the small hepatocytes aggregate in a cryopreservation solution;
(iv) transferring the suspended small hepatocyte aggregate into a freezing tube and freezing said suspended small hepatocyte aggregate; and
(v) cryopreserving the frozen small hepatocyte aggregate.

4. The method of cryopreserving small hepatocytes according to claim 3, wherein the freezing step was conducted in two steps wherein small hepatocytes are frozen at about -10°C to about -30°C, then at about -50°C to -90°C or in liquid nitrogen, and the cryopreservation of said small hepatocytes are conducted at -50°C to -90°C or in liquid nitrogen.

5. The method of cryopreservation of the small hepatocytes according to claim 3 or 4, wherein the dissociation of small hepatocytes aggregate from culture dishes is nonenzymatically performed.

6. The method of preparing the small hepatocytes according to claim 1 or 2, wherein the small hepatocytes are human small hepatocytes.

7. The method of cryopreservation according to any one of claims 3 to 5, wherein the small hepatocytes are human small hepatocytes.

8. Small hepatocytes which are cryopreserved by the method according to any one of claims 3 to 5.
